# EUROPEAN PATENT SPECIFICATION

(11) **EP 3 716 785 B1**
(45) Date of publication and mention of the grant of the patent: **29.12.2021**
(21) Application number: 18808016.2
(22) Date of filing: 29.11.2018
(51) Int. Cl.: A23L 3/3409, A23L 3/04, A23L 3/02, B65B 55/02, C12H 1/20, C09F 9/00, A61L 2/22, C02F 1/42, C02F 1/28, C02F 1/44, C02F 103/32, C02F 101/34

(54) **PASTEURIZING DEVICE AND METHOD FOR OPERATING A PASTEURIZING DEVICE**
PASTEURISIERUNGSVORRICHTUNG UND VERFAHREN ZUM BETRIEB EINER PASTEURISIERUNGSVORRICHTUNG
DISPOSITIF DE PASTEURISATION ET PROCÉDÉ DE FONCTIONNEMENT D'UN DISPOSITIF DE PASTEURISATION

(30) Priority: 30.11.2017 EP 17204731; 30.11.2017 US 201715827013
(43) Date of publication of application: 07.10.2020
(73) Proprietor: Red Bull GmbH, 5330 Fuschl am See (AT)
(72) Inventor: HAMMERER, Dietmar, 6922 Wolfurt (AT); CONCIN, Roland, 5330 Fuschl am See (AT); RINDERER, Christian, 5330 Fuschl am See (AT)
(74) Representative: Burger, Hannes
(86) International application number: PCT/EP2018/083016
(87) International publication number: WO 2019/106092

(56) References cited:
- WO-A1-2006/031957
- DE-A1-102008 017 524
- US-A1- 2009 123 330
- US-A1- 2015 368 135

## Description

Pasteurization of food is commonly used in the food producing industry to extend the shelf life of foodstuff. A widely-used method of pasteurization is to heat the foodstuff up to elevated temperatures for a certain time period. Often the foodstuff is filled into containers prior to pasteurization, and the containers are sealed. The sealed containers filled with the foodstuff are then subjected to temperature treatment for pasteurizing. Typically, this is accomplished by treating the containers with a temperature-controlled process liquid in a treatment area, for example by dispensing or spraying such temperature-controlled, aqueous process liquid onto the containers in various heating and cooling zones of the treatment area.

The process liquid or process water is commonly reused for heating and cooling the containers continuously, in order to minimize the need for fresh water and to provide for better energy efficiency of pasteurizing devices. During such continuing reuse of the process liquid, an insertion of particle and soluble contaminations into the process liquid cannot be prevented. This includes microorganism populations constantly brought in and growing in the process liquid. To keep the process liquid usable and for stabilization purposes, it is known to insert chemicals into the process liquid. Such chemicals may for example include biocides to keep microorganism population low, or surfactants or other chemicals useful for preventing the process liquid from becoming turbid. Other chemicals are used for controlling water hardness or the pH value of the process liquid for example.

In addition, it is also known to use chemicals for the treatment of the containers themselves in the treatment area of a pasteurizing device. Such chemicals may for example include cleaning agents or biocides. Other chemicals may be used to impede tarnishing or discoloration of an outside of the containers during the treatment with process liquid. As is known, metal surfaces, especially aluminium surfaces for example, show a marked tendency for discoloration or stain when exposed to hot, aqueous liquids for extended time periods. While such discoloration does in principle not impair the functionality of the containers, customers often are not willing to buy such discolored products, as they are perceived unappealing in appearance and low quality. As many containers for the storage of foodstuff comprise metal surfaces, such as aluminium cans or bottles with screw caps made of metal materials, such discoloration may lead to high amounts of products with limited or even no commercial usability.

Chemicals for the treatment of the containers themselves are nowadays also inserted into the process liquid, and are transported into the treatment area of a pasteurizer by means of the process liquid. Within the treatment area such chemicals may be evaporated, for example due to the high temperatures within the treatment area or by means of other chemicals, such as strong acids used for expelling weaker treatment acids out of the process liquid.

However, using the process liquid to transport treatment chemicals for container-treatment into the treatment area of pasteurizing devices gives rise to problems related to the chemical composition of the process liquid itself, as such treatment chemicals may interfere with other chemical compounds in the process liquid. Furthermore, the large number and large amounts of chemicals being inserted into process liquids of pasteurizing devices, also gives rise to problems associated with components of the pasteurizing devices or with the containers being temperature-treated in the pasteurizing devices, as both components of the pasteurizing devices as well as the containers may be harmed due to the exposure to process liquid containing large amounts of chemicals. Considered as a whole, management and handling of chemicals in pasteurizing devices is difficult with the methods applied nowadays.

DE 10 2008 017524 A1 discloses a device for pasteurization of foodstuff in closed containers by applying tempered treatment liquid onto the containers in one or more treatment zone(s). For disinfection purposes, the pasteurization device further comprises a dosage system for applying ozone as biocide into the treatment zone(s) in order to kill harmful microorganisms on the container and pasteurization device surfaces. The ozone is transferred into the treatment zone(s) dissolved in a transport liquid such as the treatment water, wherein the ozone degasses from the transport liquid concentrate within the treatment zone(s).

US 2015/368135 A1 also discloses a pasteurization system with a feed and evacuation conveyor system for containers filled with food. The system comprises a closed-loop circuit for re-use of the process liquid used for spraying the containers in one or more treatment zone(s). The treatment zone(s) comprise screening units for deposition of sediment from the process liquid, wherein the collected sediment can be transferred to a central filter unit and be filtered. For sanitation of the pasteurization system, US 2015/368135 A1 mentions a dosage unit designed to add a non-specific biocide to the process liquid filtered by the central filter unit.

US 2014/219994 A1 discloses sterilization and pasteurization applications. US 2014/219994 A1 teaches to add concentrated compositions/blends of chemicals comprising non-volatile organic acids with high boiling temperatures, in particular organic acids comprising multiple acid groups as corrosion inhibitors to protect the sterilizing equipment and the cans treated therewith. The chemical compositions further comprise oligomeric and/or polymeric substances. US 2014/219994 A1 teaches to add concentrated solutions of the chemical substances into the process water, wherein the concentrated solution is diluted by the process water and may be further diluted by steam upon condensation into the process water, if such steam is used to directly heat the process water.

The objective of the invention is to provide an improved method for operating a pasteurizing device, which provides for more efficient chemicals management in the pasteurizing device.

This objective is achieved by a method for operating a pasteurizing device.

The method for operating a pasteurizing device comprises a first operating condition.

In the first operating condition sealed containers filled with foodstuff are conveyed through a treatment area comprising at least one heating zone.

In the first operating condition, the foodstuff is heated and pasteurized in the at least one heating zone by dispensing a temperature-controlled process liquid onto the containers.

In the first operating condition, at least a major part of the process liquid from the treatment area is returned to the treatment area in a closed-loop circuit for reuse.

The method further comprises, that at least one treatment chemical for container-treatment is introduced into the treatment area in a gaseous or aerosolized state by means of a feed device in any operating condition of the pasteurizing device.

Through these measures, the at least one treatment chemical for container-treatment can directly be transferred to its place of application, without the need for transporting it by means of the process liquid. Therefore, the number and amount of chemicals in the process liquid may be reduced, which in turn provides for a better management of the chemical composition of the process liquid itself. In particular, any undesirable interference of treatment chemicals with other chemicals in the process liquid may be avoided.

Furthermore, the containers as well as components of the pasteurizing device itself, especially components in the treatment area are better protected, as long-term contact with a process liquid containing a large number and large amounts of chemicals can be impeded. As a result, damage to components can be impeded, and the time periods between cleaning or maintenance operations for components of a pasteurizing device may at least be prolonged.

In addition, the overall quantities of treatment chemicals for container-treatment can be lowered in comparison to the prior art, as the treatment chemicals do not need to be inserted into the process liquid in an amount in excess of actual the needed amount. When transported into the treatment area by means of the process liquid according to the prior art, excess amounts of treatment chemicals are necessary, as only a portion of the process liquid is in the treatment area at any given time. By introducing the at least one treatment chemical into the treatment area in a gaseous or aerosolized state by means of the feed device, the quantity of the at least one treatment chemical can be better controlled, and can be more precisely adjusted to the actual needs. In this way, also recommendations or requirements concerning health matters of the employees, like for example maximum allowable concentration (MAC) values or similar recommendations or requirements, can be satisfied better.

By way of the specified measures, the introduction of the at least one treatment chemical into the treatment area can also be performed in operating conditions of the pasteurizing device, where no process liquid is dispensed into the at least one heating zone of the treatment area. Therefore, an introduction of treatment chemicals into the treatment area is possible in any operating condition of the pasteurizing plant. This is for example useful in operating conditions, in which transport means for transporting the containers through the treatment area and the dispensing of process liquid into the treatment area have stopped, for example due to a failure. In such cases, undesirable changes to the containers, like discoloration of metal surfaces are likely to occur, due to the prolonged stay of the containers in the atmosphere in the treatment area. By way of the given measures, such undesired changes can be impeded during any operating condition of the pasteurizing plant. The at least one treatment chemical may dissolve into the process liquid in the treatment area after container-treatment.

An embodiment of the method for operating a pasteurizing device may comprise, that the at least one treatment chemical is introduced into the treatment area by means of a carrier gas flow. This measure provides a convenient way for introducing the at least one treatment chemical into the treatment area. By way of the carrier gas, the at least one treatment chemical can be distributed in the treatment area in diluted form.

In another embodiment, it may be convenient, when the at least one treatment chemical is vaporized prior to introduction into the treatment area. By way of this measure, also treatment chemicals, which are non-gaseous under standard temperature and pressure (STP) conditions, may be introduced into the treatment area in vaporized from.

An embodiment of the method may comprise, that the at least one treatment chemical is evaporated into a carrier gas prior to introduction into the treatment area, and subsequently is introduced into the treatment area by means of the carrier gas. This embodiment allows for introduction of especially low amounts of treatment chemical into the treatment area. In addition, the quantity of treatment chemical introduced into the treatment area can be controlled with high accuracy.

It may also be convenient, when the vaporizing is conducted or supported by means of a heating device. In this way, higher amounts of treatment chemicals can be introduced into the treatment area more quickly.

In an embodiment of the method, it may be provided, that the vaporizing is conducted or supported by means of applying a vacuum. Conducting or supporting the vaporization by means of applying a vacuum is an efficient way for vaporizing treatment chemicals or supporting the vaporization of treatment chemicals. In general, this variant allows for good control over the amount of treatment chemicals introduced into the treatment area, wherein very small amounts can be vaporized and introduced into the treatment area of the pasteurizing device. In addition, treatment chemicals sensitive to higher temperatures can be vaporized efficiently in this way, and decomposition of such chemicals can be impeded.

Another embodiment may comprise, that the at least one treatment chemical is nebulized into the treatment area, or that the at least one treatment chemical is nebulized prior to introduction into the treatment area by means of an aerosolizing device. In this way, the at least one treatment chemical can be introduced into the treatment area with high energy efficiency.

In a preferred embodiment, it may be provided, that an amount of the at least one treatment chemical introduced into the treatment area is controlled by means of at least one metering device. This allows for precise control of the quantity of treatment chemicals introduced into the treatment area. The at least one metering device may be of volumetric type for example, wherein metering of treatment chemicals may be carried out for example by flow control of the treatment chemicals, or by metering of absolute volumetric quantities of treatment chemicals for introduction into the treatment area.

Another embodiment may comprise, that that the at least one treatment chemical is introduced into the treatment area during a second operating condition, wherein in the second operating condition the conveying of the containers and the dispensing of temperature-controlled liquid onto the containers is or has stopped. This embodiment is especially useful to impede undesirable changes to the containers, for example discoloration or stain of the containers, in case of a stop of pasteurization treatment, for example due to a failure. Advantageously the at least one treatment chemical may be introduced into the treatment area although the dispensing of process liquid onto the containers in the treatment area is or has stopped.

In the method, sealed containers comprising a metal material on an exterior of the containers are conveyed through the treatment area in the first operating condition. In a more specific embodiment containers comprising aluminum on the exterior may be conveyed through the treatment area.

The method provides, that the at least one treatment chemical introduced into the treatment area comprises a volatile carboxylic acid.

A more specific embodiment may comprise, that the at least one treatment chemical introduced into the treatment area comprises formic acid or acetic acid. These treatment chemicals have proven to be effective for impeding discoloration or stain on metal materials, especially aluminium comprising containers in pasteurizing devices.

Another embodiment of the method may comprise, that at least in the first operating condition a partial quantity of the process liquid is continuously taken out of the closed-loop circuit per time unit, and is purified by means of a membrane filtration device and subsequently by means of an ion exchanger device and/or by means of an adsorption device, and the purified process liquid is returned into the treatment area. Such procedure is particularly useful for keeping the process liquid clear of particles, including microorganism, as well as removing soluble chemicals out of the process liquid. In this way, the amount of particles, microorganisms and soluble chemicals in the process liquid can be kept low, thereby protecting the containers as well as components of the pasteurizing device. As the method includes introducing treatment chemicals into the treatment area in a gaseous or aerosolized state by means of the feed device, the removal of soluble chemicals does not interfere with the addition of the treatment chemicals. The removed soluble chemicals may include treatment chemicals dissolved into the process liquid after container-treatment, which dissolving may occur especially in temperature treatment zones with relatively low temperature of the process liquid. Advantageously, the removing of particles prior to the removal of soluble chemicals protects the ion exchanger device and/or the adsorption device from getting polluted with particles.

A pasteurizing device comprises a treatment area configured for temperature treatment of the containers with at least one heating zone, and a conveying system for conveying the sealed containers filled with foodstuff through the treatment area.

The pasteurizing device further comprises irrigation means configured for dispensing a temperature-controlled process liquid into the at least one heating zone, and a closed-loop circuit configured for returning process liquid from the treatment area back into the treatment area for reuse.

In addition, the pasteurizing device comprises at least one feed device configured for introducing at least one treatment chemical for container-treatment in gaseous or aerosolised state into the treatment area.

By way of these features, treatment chemicals for container-treatment can be introduced into the treatment area based on demand in any operating condition. Treatment chemicals for container-treatment can directly be transferred to their place of application, without the need for transport by means of the process liquid.

This allows for better protection of the containers during operation of the pasteurizing device, as well as for better protection of components of the pasteurizing device itself, as a long-term contact with a process liquid containing a large number and large amounts of chemicals can be impeded during operation of the pasteurizing device.

The possibility of introducing the at least one treatment chemical into the treatment area in a gaseous or aerosolized state by means of the feed device, also enables better control over the quantity of the at least one treatment chemical introduced, and a more precise adjustment to the actual needs. In this way, also recommendations or requirements concerning health matters of the employees, like for example maximum allowable concentration (MAC) values or similar recommendations or requirements, can be satisfied better.

An embodiment of the pasteurizing device may comprise, that the feed device comprises a means for generating a carrier gas flow, and that the feed device is configured for transporting the at least one treatment chemical into the treatment area by means of the carrier gas flow. By way of this features treatment chemicals can be introduced into the treatment area by means of the carrier gas flow.

Another embodiment of the pasteurizing device may comprise, that the feed device is configured for guiding the carrier gas through an evaporating space containing the at least one treatment chemical, and subsequently into the treatment area. The evaporating space containing the at least one chemical may for example be an evaporator comprising means for supporting the evaporation of the at least one treatment chemical, or simply a reservoir containing the at least one treatment chemical. In this way, the at least one treatment chemical can be evaporated into the carrier gas prior to introduction into the treatment area, and subsequently can be introduced into the treatment area by means of the carrier gas.

In yet another embodiment it may be provided, that the feed device comprises a heating device configured for vaporizing the at least one treatment chemical or for supporting vaporization of the treatment chemical. By way of these features, the at least one treatment chemical may be vaporized and introduced in into the treatment area directly in gaseous form, or an evaporation of the at least one treatment chemical, for example into a carrier gas may be supported by using the heating device.

A further embodiment of the pasteurizing device may comprise, that the feed device comprises a vacuum device configured for vaporizing the at least one treatment chemical or for supporting vaporization of the at least one treatment chemical. This feature allows for vaporization or for supporting vaporization of the at least one treatment chemical by applying a vacuum. In particular, treatment chemicals sensitive to higher temperatures can be vaporized efficiently in this way, and decomposition of such chemicals can be impeded.

It may also be expedient, if the feed device comprises at least one aerosolizing means configured for nebulizing the at least one treatment chemical into the treatment area. Such aerosolizing means may for example be atomizing nozzles arranged at or in the treatment area. By way of such aerosolizing means, treatment chemicals can be nebulized directly into the treatment area of the pasteurizing device.

A variant of the pasteurizing device may comprise, that the feed device comprises an aerosolizing device configured for nebulizing the at least one treatment chemical prior to introduction into the treatment area. Such aerosolizing devices may for example be of vibrational or rotational type, or ultrasonic atomizers.

A preferred embodiment of the pasteurizing device may comprise, that the feed device comprises at least one metering device configured for controlling an amount of treatment chemical introduced into the treatment area. This feature allows for precise control of the quantity of treatment chemicals introduced into the treatment area. The at least one metering device may be of volumetric type for example, wherein metering of treatment chemicals may be carried out for example by flow control of the treatment chemicals, or by metering of absolute volumetric quantities of treatment chemicals for introduction into the treatment area. The metering device may be configured for metering liquid treatment chemicals or gaseous, respectively already vaporized treatment chemicals. When the metering device is configured for metering gaseous treatment chemicals, the metering device may for example be a mass-flow controller.

In another embodiment, it may be provided, that the feed device comprises feed orifices arranged in different temperature treatment zones of the treatment area. By way of these features, the at least one treatment chemical can be introduced based on demand into different temperature treatment zones, for example heating zones, pasteurizing zones and cooling zones.

Furthermore, it may be expedient, that at least one shut-off device is associated with a feed orifice, or with a group of feed orifices. By way of these features, the at least one treatment chemical can be introduced into specific zones of the pasteurizing device, based on demand. A treatment chemical may for example be introduced into a zone with high probability for discoloration of containers comprising a metal material, while other temperature treatment zones of the pasteurizing may not be fed with the corresponding treatment chemical.

In another embodiment of the pasteurizing device it may provided, that the pasteurizing device comprises at least one removing means configured for removal of a partial quantity of the process liquid from the closed-loop circuit per time unit, and a purification device comprising a membrane filtration device and an ion exchanger device and/or an adsorption device downstream of the membrane filtration device configured for cleaning the removed partial quantity of the process liquid into, and at least one returning means configured for returning the purified process liquid back into the treatment area. During normal operation of the pasteurizing device, a partial quantity of the process liquid can continuously be taken out of the closed-loop circuit and be purified by means of a membrane filtration device and subsequently by means of an ion exchanger device and/or by means of an adsorption device, and the purified process liquid can be returned into the treatment area.

To provide a better understanding, the invention is described in more detail in the following with reference to the appended figures.

These appended figures are highly simplified, schematic drawings illustrating the following:
- Fig. 1: An exemplary embodiment of a pasteurizing device;
- Fig. 2: an exemplary embodiment of a feed device configured for introducing at least one treatment chemical in gaseous state into a treatment are of a pasteurizing device;
- Fig. 3: another exemplary embodiment of a feed device configured for introducing at least one treatment chemical in gaseous state into a treatment are of a pasteurizing device;
- Fig. 4: another exemplary embodiment of a feed device configured for introducing at least one treatment chemical in gaseous state into a treatment are of a pasteurizing device;
- Fig. 5: another exemplary embodiment of a feed device comprising an aerosolizing means;
- Fig. 6: another exemplary embodiment of a feed device comprising an aerosolizing means.

Introductory, it should be pointed out, that the same parts described in the different embodiments are denoted by the same reference numbers and the same component names and the disclosures made throughout the description can be transposed in terms of meaning to same parts bearing the same reference numbers or same component names. Furthermore, the positions chosen for the purposes of the description, such as top, bottom, side, etc., relate to the drawing specifically being described and can be transposed in terms of meaning to a new position when another position is being described.

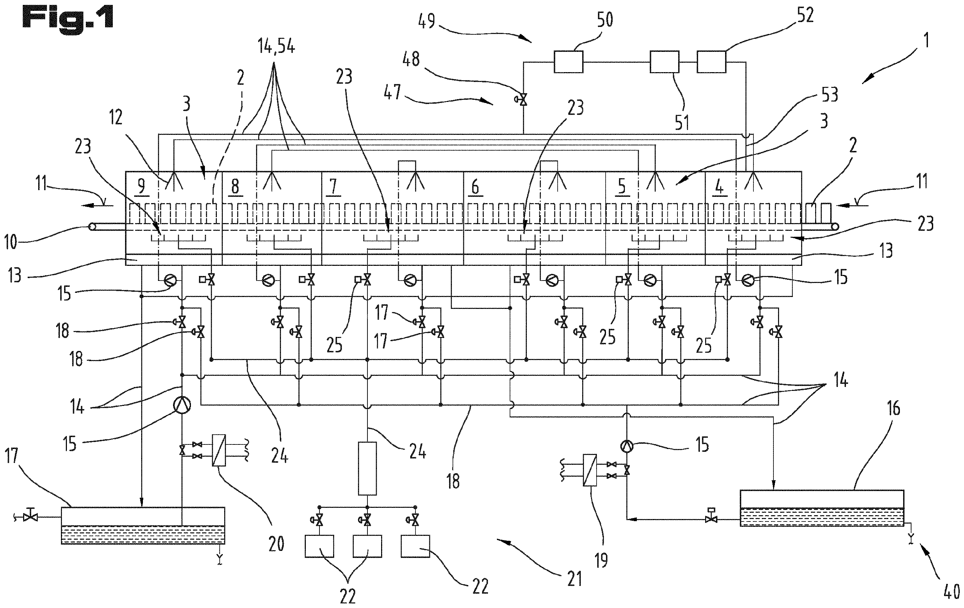

Fig.1 schematically shows an exemplary embodiment of a pasteurizing device 1 for pasteurizing foodstuff in sealed containers 2. The pasteurizing device 1 is configured as so-called tunnel pasteurizer, and comprises a treatment area 3 configured for temperature treatment of the containers with at least one heating zone 4. The treatment area 3 of the pasteurizing device 1 shown in Fig. 1 as example comprises a second heating zone 5 for further heating up the foodstuff, two heating zones 6, 7 for pasteurizing the foodstuff in the containers 2, and two cooling zones 8, 9 for cooling down the foodstuff. Depending on need or purpose, it is of course possible for a pasteurizing device 1 to comprise more or fewer zones 3, 4, 5, 6, 7, 8, 9 as compared to the exemplary embodiment shown in Fig. 1. It is likewise possible for further treatment zones to be provided in the treatment area 3. For example, a zone for drying the outer side of the containers 2 could be provided after the last cooling zone 9. For reasons of clarity, such alternative designs of pasteurizing devices are not described. A person skilled in the art will appreciate, that the present invention implies such alternative designs of pasteurizing devices or treatment areas, respectively.

The example for a pasteurizing device 1 shown in Fig. 1 comprises a conveying system 10 for conveying the sealed containers 2 filled with foodstuff through the treatment area 3 in a transport direction 11. The conveying system may for example comprise a conveyor belt, which is pervious liquids and gases. The pasteurizing device 1 further comprises irrigation means 12 configured for dispensing a temperature-controlled process liquid into the temperature treatment zones 4, 5, 6, 7, 8, 9 of the treatment area 3. The irrigation means 12 may for example be sprinkler or shower devices known in the art, configured for dispensing the respective temperature-controlled process liquid onto the containers 2.

In a first or normal operation condition of the exemplary pasteurizing device 1 shown in Fig. 1, sealed containers 2 filled with foodstuff are conveyed through the treatment area 3 comprising the heating zones 4, 5, 6, 7 and the cooling zones 8, 9. In the example shown in Fig. 1 the foodstuff is heated and pasteurized in two heating zones 6, 7 by dispensing a temperature-controlled process liquid onto the containers 2. For the purpose of pasteurizing the foodstuff, a temperature of the process liquid dispensed onto the containers 2 in heating zones 6, 7 is selected high enough for pasteurization. These heating zones 6, 7 may also be referred to as pasteurizing zones 6, 7. In the exemplary embodiment of the pasteurizing device 1 shown in Fig. 1, the foodstuff in the containers 2 is heated up in a controlled manner in the heating zones 4, 5, prior to pasteurization in heating zones 6, 7. Typically a temperature of the process liquid dispensed into these pre-heating zones 4, 5 will be growing from heating zone 4 to heating zone 5. After having been temperature-treated in the heating zones 4, 5, 6, 7, the foodstuff in the containers 2, is then cooled down in a controlled manner by dispensing process liquid with a temperature low enough for cooling the containers 2 in cooling zones 8, 9.

After having crossed the respective temperature treatment zones 4, 5, 6, 7, 8, 9 the process liquid may be collected at bottom areas 13 of the treatment zones 4, 5, 6, 7, 8, 9, wherein at least a major part of the process liquid from the treatment area 3 is returned to the treatment area 3 for reuse. For this purpose, the pasteurizing device 1 comprises a closed-loop circuit 14 configured for returning process liquid from the treatment area 3 back into the treatment area 3 for reuse. For reusing the process liquid, the pasteurizing device 1 may comprise recuperation lines 54, arranged for transporting process liquid collected in the bottom areas 13 of temperature treatment zones 4, 5, 8, 9 directly back into different temperature treatment zones 4, 5, 8, 9, as can be depicted from the example for a pasteurizing device 1 shown in Fig. 1. In the first operating condition, at least a portion of the process liquid from the bottom area 13 of the last cooling zone 9 may for example be transferred to the first heating zone 4, and vice versa. Such recuperation of process liquid is particularly meaningful with the pasteurizing device 1 shown as example in Fig. 1, as the temperature of the process liquid falls upon heating up the containers 2 in the heating zones 4, 5, and rises upon cooling down the containers 2 in the cooling zones 8, 9. Circulation devices 15, for example circulation pumps may be used to convey the process liquid in the closed-loop circuit 14, as is shown in Fig. 1.

Portions of process liquid with comparatively high temperature level collected at the bottom areas 13, may be transferred to a hot collection tank 16 of the pasteurizing device 1 for example. Portions of process liquid with comparatively low temperature level collected at the bottom areas 13, may be transferred to a cold collection tank 17. The process liquids in the hot and cold collection tanks 16, 17 may be used to set or adjust respective temperatures for process liquid to be fed into the individual temperature treatment zones 4, 5, 6, 7, 8, 9. For this purpose, each of the circulation devices 15 on an input side may be connected with the hot and cold collection tanks 16, 17 via metering valves 18, as shown in the embodiment according to Fig. 1. In this way, process liquid from the hot collection tank 16 and the cold collection tank 17 may be used in the closed-loop circuit 14 for temperature-adjustment of process liquid flows to be fed into one of the individual, respective temperature treatment zones 4, 5, 6, 7, 8, 9 of the pasteurizing device 1, by adding certain amounts of process liquid from the hot and cold collection tanks 16, 17 in a controlled manner.

For heating up the process liquid in the pasteurizing device 1, a heating means 19 may be provided, for cooling the process liquid a cooling means 20 may be provided. The heating means 19 and the cooling means 20 may both for example be heat exchangers, wherein a primary side of the heat exchangers 19, 20 may be connected with a heating device (not shown) and a cooling device (not shown) respectively. A water steam generator may be used as heating device for example, an air- or water-cooled cooling tower may be used as cooling device. Alternatively or in addition, it may be expedient to use a heat pump as heating and cooling device.

As is shown in Fig. 1, the pasteurizing device 1 comprises at least one feed device 21 configured for introducing at least one treatment chemical for container-treatment in gaseous or aerosolised state into the treatment area 3. By means of the feed device 21, at least one treatment chemical for container-treatment can be introduced into the treatment area 3 in a gaseous or aerosolized state. The introduction of the treatment chemical into the treatment area 3 can be conducted based on demand and in any operating condition of the pasteurizing device 1, as the introduction is independent from whether process liquid is dispensed into the treatment area 3, respectively the temperature-treatment zones 4, 5, 6, 7, 8, 9, or not.

The feed device 21 may for example comprise reservoirs 22 containing treatment chemicals. A treatment chemical may be a gas under standard temperature and pressure (STP) conditions. In such case, a gaseous treatment chemical may be introduced into the treatment area 3 directly from the corresponding reservoir 22, for example in pure form or diluted in a carrier gas. In many cases, suitable treatment chemicals are not gaseous under STP conditions however, so that the feed device 21 may comprise means for vaporizing or aerosolizing uch treatment chemicals. Some embodiments of the feed device 21 comprising such means for vaporizing or aerosolizing treatment chemicals will be described in the following with reference to the figures.

Irrespective therefrom, the feed device 21 may comprise feed orifices 23 arranged at or in different temperature treatment zones 4, 5, 6, 7, 8, 9 of the treatment area 3, and feed lines 24 for supplying the at least one treatment chemical to the feed orifices 23 for introduction of the at least one treatment chemical into the treatment area 3, respectively the temperature-treatment zones 4, 5, 6, 7, 8, 9. Furthermore, at least one shut-off device 25 may be associated with a feed orifice 23, or with a group of feed orifices 23, such that the at least one treatment chemical may be introduced into only specific temperature-treatment zones 4, 5, 6, 7, 8, 9 of the pasteurizing device 1 for example. A shut-off device 25 as shown in Fig. 1 may for example be an open/close valve, or a metering valve.

In the example shown in Fig. 1, feed orifices 23 are arranged in or at all of the temperature treatment zones 4, 5, 6, 7, 8, 9, such that the whole treatment area 3 can be supplied with treatment chemical(s). In alternative embodiments, feed orifices 23 may be arranged in or at specific temperature treatment zones 4, 5, 6, 7, 8, 9 only. The feed orifices 23 may for example be arranged in the treatment area 3 underneath the pervious conveyor belt 10 for introducing the gaseous or aerosolized treatment chemical to an area below the containers 2. Other arrangements of the feed orifices 23 are also possible, for example an arrangement which allows for applying the at least one treatment chemical from multiple sides onto the containers 2.

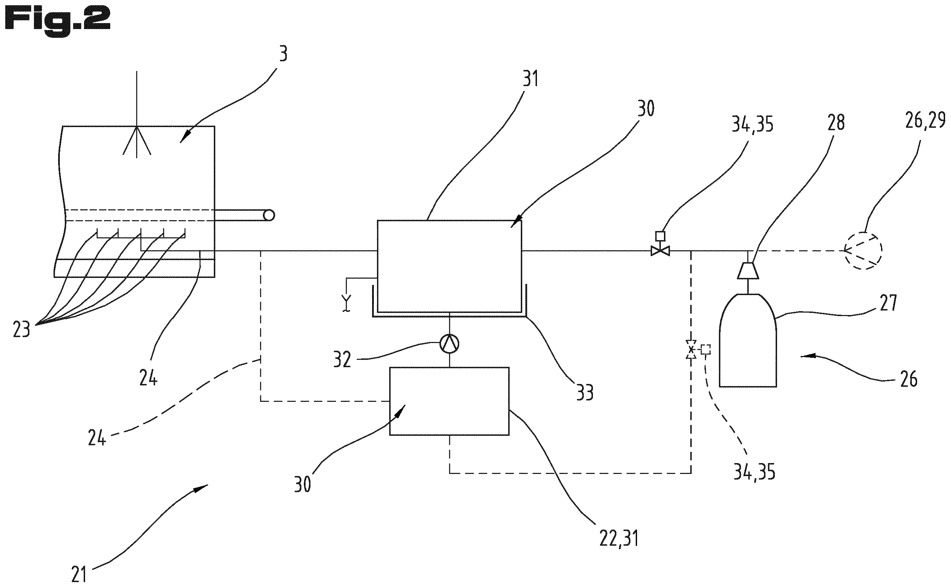

Fig. 2 shows a schematic view of an embodiment of a feed device 21 for introducing at least one treatment chemical in gaseous state into the treatment area 3, in more detail. The same reference numbers and component names are used in Fig. 2 to denote parts that are the same as those described above in connection with Fig. 1. To avoid unnecessary repetition, reference may be made to the more detailed description of Fig. 1 given above. As can be depicted from Fig. 2, the feed device 21 may comprise a means 26 for generating a carrier gas flow. In the exemplary embodiment shown in Fig. 2 the feed device 21 is configured for transporting the at least one treatment chemical into the treatment area 3 by means of the carrier gas flow.

The means 26 for generating the carrier gas flow, may for example be a pressure vessel 27 filled with the carrier gas, and equipped with a throttle valve 28. The carrier gas within the pressure vessel may be air or an inert gas like nitrogen or argon for example. Alternatively, a small-sized, driven fan 29 or any other airflow generating means may be used the generate the carrier gas flow, as indicated with dashed lines in Fig. 2. With the features of the exemplary embodiment of the feed device 21 shown in Fig. 2, the at least one treatment chemical can be introduced into the treatment area 3 by means of the carrier gas flow.

In case the at least one treatment chemical is not gaseous under STP conditions, the at least one treatment chemical may be vaporized prior to introduction into the treatment area 3. For example, the at least one treatment chemical may be evaporated into the carrier gas prior to introduction into the treatment area 3, and subsequently may be introduced into the treatment area 3 by means of the carrier gas. The embodiment of the feed device 21 shown in Fig. 2 is configured for guiding the carrier gas through an evaporating space 30 containing the at least one treatment chemical, and subsequently into the treatment area 3.

For this purpose, the at least one treatment chemical may for example be pumped from a reservoir 22 into an evaporating space 30 of an evaporator 31 by means of a liquid pump 32. The feed device 21 may be configured to guide the carrier gas through the liquid treatment chemical, such that the evaporator 31 is configured as throughflow evaporator in this case. Alternatively the feed device 21 may be configured to guide the carrier gas through a gas space over the liquid treatment chemical, such that the evaporator 31 is configured as surface evaporator. As is shown in Fig. 2, the feed device 21 may comprise a heating device 33 for supporting the evaporation of the at least one chemical into the carrier gas.

In an alternative embodiment shown in dashed lines in Fig. 2, the feed device 21 may be configured for guiding the carrier gas directly through an evaporating space 30 of the reservoir 22 containing the treatment chemical. In such case the reservoir 21 is configured as evaporator31 itself. Either way, the carrier gas may be guided through an evaporating space 30, wherein the at least one treatment chemical is evaporated into the carrier gas, and the carrier gas saturated with the treatment chemical is then introduced into the treatment area 3 via the feed line 24 and feed orifices 23.

With the exemplary embodiment of the feed device 21 shown in Fig. 2, the amount of treatment chemical introduced into the treatment area 3 can in principle be controlled manually or automatically by engaging liquid pump 32 and/or engaging the carrier gas flow for a certain time period. Preferably, the amount or quantity of treatment chemical introduced into the treatment area 3 is controlled automatically by at least one metering device 34. The feed device 21 shown as example in Fig. 2 comprises at least one metering device 34 configured for controlling the amount of treatment chemical introduced into the treatment area 3.

In the exemplary embodiment shown in Fig. 2, the at least one metering device 34 may be a mass or volumetric gas flow controller 35 for example. In this case, the concentration of treatment chemical in the carrier gas flow after having passed through the evaporating space 30 can in principle be measured by suitable measuring devices, or can be calculated in advance by using the known temperature and the known throughput rate of the carrier gas through the evaporator 31. By means of such gas flow controller 35, the amount or quantity of treatment chemical introduced into the treatment area 3 can then be controlled by means of the amount of carrier gas introduced into the treatment area 3.

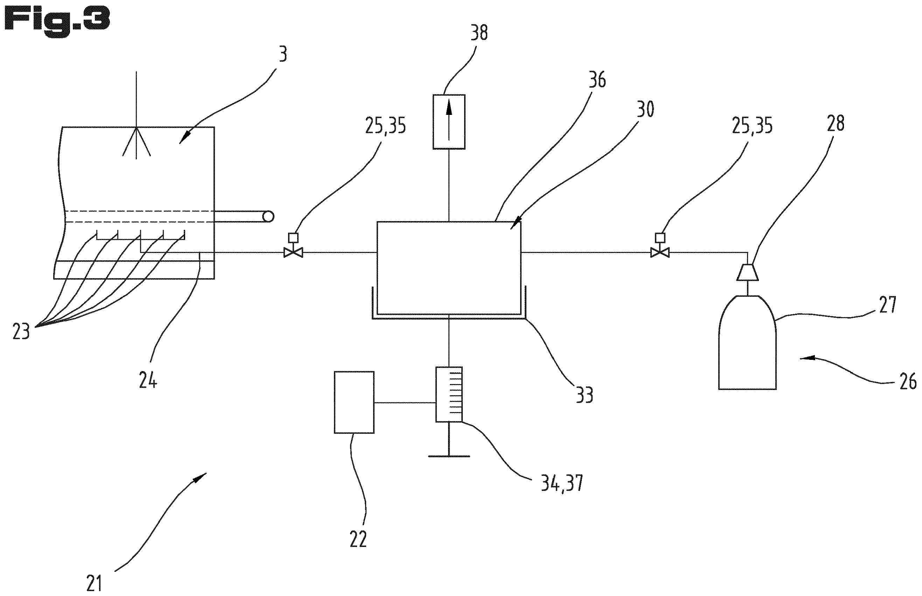

Fig. 3 shows another example of a feed device 21 configured for introducing at least one treatment chemical for container-treatment in gaseous state into the treatment area 3. The same reference numbers and component names are used in Fig. 3 to denote parts that are the same as those described above in connection with Fig. 1 and Fig. 2. To avoid unnecessary repetition, reference may be made to the more detailed description of Fig. 1 and Fig. 2 given above.

The example shown in Fig. 3 also comprises a reservoir 22 and a means 26 for generating a carrier gas flow. The exemplary feed device 21 shown in Fig. 3 comprises a vaporizer 36 for vaporizing the at least one treatment chemical. In case of the example shown in Fig. 3, a limited amount or quantity of liquid treatment chemical may be fed into the vaporizer 36 from the reservoir 22 by means of a metering device 34. The metering device 34 may be a liquid metering pump 37, like a small piston metering pump, or a peristaltic pump for example. The liquid treatment chemical may then for example vaporized in the vaporizer 36 by means of a heating device 33 prior to introduction into the treatment area 3 by means of the carrier gas flow, or the heating means 33 may be used to support evaporation of the treatment chemical into the carrier gas flow within the vaporizer 36.

As shown in Fig. 3, the feed device 21 may comprise a vacuum device 38 configured for vaporizing the at least one treatment chemical or for supporting vaporization of the at least one treatment chemical. The vaporizing of the at least one treatment chemical may be conducted or supported by means of applying a vacuum in this way. The feed device 21 may comprise shut-off devices 25, for allowing or prohibiting the carrier gas from flowing through the vaporizer 36. As can be depicted from Fig. 3, the vaporizer 36 can be shut off from the means 26 for generating the carrier gas flow, as well as from the treatment area 3. In closed position of the shut-off means 25, a certain amount of liquid treatment chemical can be introduced into the vaporizer 36 from the reservoir 21. The liquid treatment chemical may then be vaporized in the vaporizer 36 by means of the vacuum device 38, or by means of the heating device 33, or by means of both the vacuum device 38 and the heating device 33.

After vaporizing the treatment chemical, the shut-off devices 25 can then be opened, to allow carrier gas to flow through the vaporizer 36, and carry the vaporized treatment chemical into the treatment area 3. The shut-off devices 25 may also be configured as gas flow controllers 35, in order to provide a means for controlling the amount of carrier gas and treatment chemical introduced into the treatment area 3 per time unit.

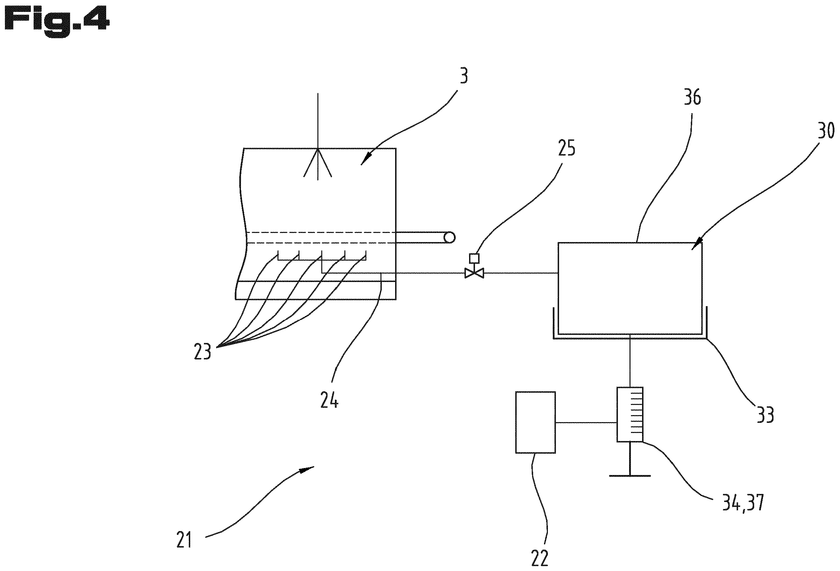

In Fig. 4, another example of a feed device 21 configured for introducing at least one treatment chemical for container-treatment in gaseous state into the treatment area 3 is shown. The same reference numbers and component names are used in Fig. 4 to denote parts that are the same as those described above in connection with Fig. 1 to Fig. 3. To avoid unnecessary repetition, reference may be made to the more detailed description of Fig. 1 to Fig. 3 given above.

Fig. 4 shows an embodiment, which is especially suited for introducing relatively high amounts of treatment chemical in gaseous state into the treatment area 3, per time unit. The feed device 21 comprises a reservoir 22 for a liquid treatment chemical and vaporizer 36 with a heating device 33 for vaporizing the liquid treatment chemical. In case of the example shown in Fig. 4, no carrier gas flow is used to introduce the treatment chemical into the treatment area 3. The treatment chemical is directly vaporized in the vaporizers 36, and enters the treatment area 3 in gaseous state due to the higher pressure generated in the vaporizer 36.

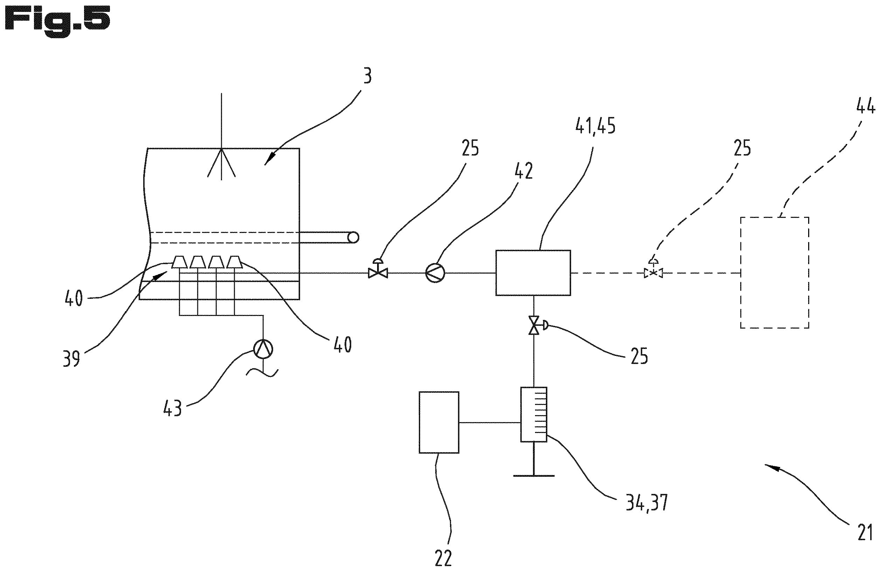

Fig. 5 shows an example of a feed device 21 configured for introducing the at least one treatment chemical for container-treatment in aerosolised state into the treatment area 3. The same reference numbers and component names are used in Fig. 5 to denote parts that are the same as those described above in connection with Fig. 1 to Fig. 4. To avoid unnecessary repetition, reference may be made to the more detailed description of Fig. 1 to Fig. 4 given above.

As shown in Fig. 5, the feed device 21 may comprise at least one aerosolizing means 39 configured for nebulizing the at least one treatment chemical into the treatment area 3. The aerosolizing means 39, may comprise atomizing or spraying nozzles 40. A controlled amount of treatment chemical may for example be pumped from a reservoir 22 into a buffer storage 41, by means of a metering device 34, for example a liquid metering pump 37. The treatment chemical may then for example be fed through the atomizing or spraying nozzles 40 by means of a high pressure pump 42, and be nebulized into the treatment area 3. Alternatively or in addition, the spraying nozzles 40 may be connected with a source of pressurized gas or air, like the compressor 43 shown in Fig. 5. The aerosolizing may then be accomplished or supported by the pressurized gas or air. With the design of the feed device 21 shown in Fig. 5, the at least one treatment chemical may be nebulized into the treatment area 3.

As also shown in Fig. 5, the feed device 21 may also comprise a reservoir 44 for an auxiliary substance for supporting nebulizing of the treatment chemical. In this case, the buffer storage 41 may be configured as mixing unit 45 for mixing the treatment chemical with the auxiliary substance, and then nebulizing the mixture into the treatment area 3 by means of the atomizing or spraying nozzles 40. The auxiliary substance may for example be water.

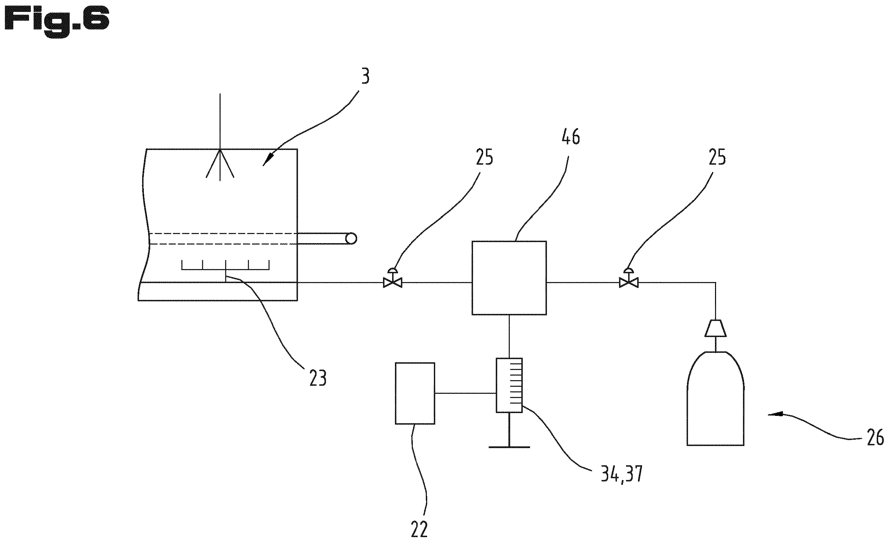

Fig. 6 shows another example of a feed device 21 configured for introducing the at least one treatment chemical for container-treatment in aerosolised state into the treatment area 3. The same reference numbers and component names are used in Fig. 6 to denote parts that are the same as those described above in connection with Fig. 1 to Fig. 5. To avoid unnecessary repetition, reference may be made to the more detailed description of Fig. 1 to Fig. 5 given above.

As shown in Fig. 6, the feed device 21 may comprise an aerosolizing device 46 configured for nebulizing the at least one treatment chemical prior to introduction into the treatment area 3. Liquid treatment chemical from the reservoir 22 may be pumped into the aerosolizing device 46 by means of a metering device 34, for example the liquid metering pump 37 shown in Fig. 6. The treatment chemical may then be aerosolized in the aerosolizing device 46, and be introduced in the aerosolized state into the treatment area 3, for example by means of a carrier gas flow generated by the carrier gas flow generating means 26. An aerosolizing device 46 may for example be of vibrational or rotational type, or an ultrasonic atomizer. With the design of the feed device 21 shown in Fig. 6, the at least one treatment chemical may be nebulized prior to introduction into the treatment area 3 by means of the aerosolizing device 46.

Independent of the structural and technical design of the feed device 21, the at least one treatment chemical may be introduced into the treatment area 3 during a second operating condition. The second operating condition may be a condition, in which the conveying of the containers 2 and the dispensing of temperature-controlled liquid onto the containers 2 is or has stopped. In such second operation condition, the containers 2, the containers may have a long residence time in the treatment area 3, which may cause unwanted changes to the containers 2. This may be due to the long time of exposure of the containers 2 to vapors and condensate of the process liquid.

In a reservoir 22, the at least one treatment chemical may be provided dissolved in a solvent, or in pure form. In principle, a treatment chemical may be provided in dilute or concentrated solutions in a reservoir 22. Based on the type of treatment chemical, different solvents may be used. The solvent may be water for example, such that an aqueous solution of the treatment chemical may be provided in a reservoir 22. If the treatment chemical is provided dissolved in a solvent, at least part of the solvent may also be introduced into the treatment area 3 in gaseous or aerosolized state. Preferably, the at least one treatment chemical is provided in pure form, or in concentrated solution in a reservoir 22.

In the first operating condition sealed containers 2 comprising a metal material on an exterior of the containers 2 are conveyed through the treatment area 3. In particular, containers 2 comprising aluminum on the exterior may be conveyed through the treatment area 3. In such cases, discoloration or stain on the outside of the containers 2 is likely to occur, when the conveying of such containers 2 through the treatment area 3 is or has stopped in the second operating condition. The conveying of the containers 2 may for example have stopped due to a failure of the conveying system 10.

In order to impede such discoloration of metal surfaces, the at least one treatment chemical introduced into the treatment area 3 comprises a volatile carboxylic acid. Such volatile organic acid may be introduced into the treatment area 3 in a gaseous or aerosolized state by means of a feed device 21 according to the embodiments shown in Fig. 2 to Fig. 6, and described above. For impeding discoloration of metal surfaces, the at least one treatment chemical introduced into the treatment area 3 may comprise formic acid or acetic acid, as these treatment chemicals have proven to be effective for this purpose.

In Fig. 1, another embodiment of the invention is shown. As can be depicted from Fig. 1, the pasteurizing device 1 may comprise at least one removing means 47 configured for removal of a partial quantity of the process liquid from the closed-loop circuit 14 per time unit. Such removing means 47 may for example comprise a T-connection and a liquid flow control valve 48, as can be depicted from Fig. 1. As is further shown in Fig. 1, the pasteurizing device 1 may comprise a purification device 49 comprising a membrane filtration device 50 and an ion exchanger device 51 and/or an adsorption device 52 downstream of the membrane filtration device 50, configured for cleaning the removed partial quantity of the process liquid. The pasteurizing device 1 may further comprise at least one returning means 53 configured for returning the purified process liquid back into the treatment area 3, which returning means 53 may for example be a simple returning line leading into one of the temperature treatment zones 4, 5, 6, 7, 8, 9 of the treatment area 3.

With such design of the pasteurizing device 1, at least in the first operating condition a partial quantity of the process liquid may be continuously taken out of the closed-loop circuit 14 per time unit, and can be purified by means of the membrane filtration device 50 and subsequently by means of an ion exchanger device 51 and/or by means of an adsorption device 52, and then the purified process liquid can be returned into the treatment area 3. Preferably the purified process liquid is returned into a temperature treatment zone 4, 5, 6, 7, 8, 9, in which the temperature of the process liquid at least roughly matches the temperature of the purified process liquid.

In this way, the amount of particles, microorganisms and soluble chemicals in the process liquid can be kept low, thereby protecting the containers 2 as well as components of the pasteurizing device 1. As the method includes introducing treatment chemicals into the treatment area 3 in a gaseous or aerosolized state by means of the feed device 21, the removal of soluble chemicals does not interfere with the addition of the treatment chemicals. The removed soluble chemicals may include treatment chemicals dissolved into the process liquid after container-treatment, which dissolving may occur especially in temperature treatment zones 4, 5, 6, 7, 8, 9 with relatively low temperature of the process liquid. Advantageously, the removing of particles prior to the removal of soluble chemicals protects the ion exchanger device 51 and/or the adsorption device 52 from getting polluted with particles.

The embodiments illustrated as examples represent possible variants and it should be pointed out at this stage that the invention is not specifically limited to the variants specifically illustrated, and instead the individual variants may be used in different combinations with one another and these possible variations lie within the reach of the person skilled in this technical field given the disclosed technical teaching.

The protective scope is defined by the claims. However, reference may be made to the description and drawings with a view to interpreting the claims. Individual features or combinations of features from the different examples of embodiments described and illustrated may also be construed as independent embodiments of the solutions proposed by the invention. The objective underlying the individual solutions proposed by the invention may be found in the description.

For the sake of good order, finally, it should be pointed out that, in order to provide a clearer understanding of the structure, elements are illustrated to a certain extent out of scale and/or on an enlarged scale and/or on a reduced scale.

## Claims

1. A method for operating a pasteurizing device (1) comprising a first operating condition, wherein
in the first operating condition sealed containers (2) filled with foodstuff are conveyed through a treatment area (3) comprising at least one heating zone (6, 7),
the foodstuff is heated and pasteurized in the at least one heating zone (6, 7) by dispensing a temperature-controlled process liquid onto the containers (2),
wherein at least a major part of the process liquid from the treatment area (3) is returned to the treatment area (3) in a closed-loop circuit (14) for reuse,
and wherein in the first operating condition sealed containers (2) comprising a metal material on an exterior of the containers (2) are conveyed through the treatment area (3),
**characterized in that**
at least one treatment chemical for container-treatment is introduced into the treatment area (3) in a gaseous or aerosolized state by means of a feed device (21) in any operating condition of the pasteurizing device (1),
wherein the at least one treatment chemical introduced into the treatment area (3) comprises a volatile carboxylic acid.

2. The method according to claim 1, **characterized in that** the at least one treatment chemical is introduced into the treatment area (3) by means of a carrier gas flow.

3. The method according to claim 1 or 2, **characterized in that** the at least one treatment chemical is vaporized prior to introduction into the treatment area (3).

4. The method according to claim 3, **characterized in that** the at least one treatment chemical is evaporated into a carrier gas prior to introduction into the treatment area (3), and subsequently is introduced into the treatment area (3) by means of the carrier gas.

5. The method according to claim 3, **characterized in that** the vaporizing is conducted or supported by means of a heating device (33).

6. The method according to claim 3 or 5, **characterized in that** the vaporizing is conducted or supported by means of applying a vacuum.

7. The method according to claim 1, **characterized in that** the at least one treatment chemical is nebulized into the treatment area (3), or that the at least one treatment chemical is nebulized prior to introduction into the treatment area (3) by means of an aerosolizing device (46).

8. The method according to any one of the preceding claims, **characterized in that** an amount of the at least one treatment chemical introduced into the treatment area (3) is controlled by means of at least one metering device (34).

9. The method according to any one of the preceding claims, **characterized in that** the at least one treatment chemical is introduced into the treatment area (3) during a second operating condition, wherein in the second operating condition the conveying of the containers (2) and the dispensing of temperature-controlled liquid onto the containers (2) is or has stopped.

10. The method according to claim 1, **characterized in that** containers (2) comprising aluminum on the exterior are conveyed through the treatment area (3).

11. The method according to claim 1, **characterized in that** the at least one treatment chemical introduced into the treatment area (3) comprises formic acid or acetic acid.

12. The method according to any one of the preceding claims, **characterized in that** at least in the first operating condition a partial quantity of the process liquid is continuously taken out of the closed-loop circuit (14) per time unit, and is purified by means of a membrane filtration device (50) and subsequently by means of an ion exchanger device (51) and/or by means of an adsorption device (52), and the purified process liquid is returned into the treatment area (3).

## Patentansprüche

1. Verfahren zum Betreiben einer Pasteurisierungsvorrichtung (1), umfassend einen ersten Betriebszustand, wobei
im ersten Betriebszustand, verschlossene Behältnisse (2), die mit Nahrungsmitteln gefüllt sind, durch einen Behandlungsbereich (3) gefördert werden, der zumindest eine Aufheizzone (6, 7) umfasst,
das Nahrungsmittel in der zumindest einen jeweiligen Aufheizzone (6, 7) durch Abgeben einer temperaturgeregelten Prozessflüssigkeit auf die Behältnisse (2) erhitzt und pasteurisiert wird,
wobei zumindest ein Großteil der Prozessflüssigkeit aus dem Behandlungsbereich (3) in einem geschlossenen Kreislauf (14) zur Wiederverwendung zum Behandlungsbereich (3) zurückgeführt wird,
und wobei im ersten Betriebszustand verschlossene Behältnisse (2), die auf einer Außenseite der Behältnisse (2) einen Metallwerkstoff umfassen, durch den Behandlungsbereich (3) gefördert werden,
**dadurch gekennzeichnet, dass**
in jedem beliebigen Betriebszustand der Pasteurisierungsvorrichtung (1) zumindest eine Behandlungschemikalie zur Behältnisbehandlung in einem gasförmigen oder aerosolierten Zustand mittels einer Zuführungsvorrichtung (21) in den Behandlungsbereich (3) eingebracht wird,
wobei die zumindest in den Behandlungsbereich (3) eingeführte eine Behandlungschemikalie eine flüchtige Carbonsäure umfasst.

2. Verfahren nach Anspruch 1, **dadurch gekennzeichnet, dass** die zumindest eine Behandlungschemikalie durch einen Trägergasstrom in den Behandlungsbereich (3) eingebracht wird.

3. Verfahren nach Anspruch 1 oder 2, **dadurch gekennzeichnet, dass** die zumindest eine Behandlungschemikalie vor dem Einbringen in den Behandlungsbereich (3) zerstäubt wird.

4. Verfahren nach Anspruch 3, **dadurch gekennzeichnet, dass** die zumindest eine Behandlungschemikalie vor dem Einbringen in den Behandlungsbereich (3) in ein Trägergas verdampft wird und in Folge mittels des Trägergases in den Behandlungsbereich (3) eingebracht wird.

5. Verfahren nach Anspruch 3, **dadurch gekennzeichnet, dass** das Verdampfen von einer Aufheizvorrichtung (33) durchgeführt oder unterstützt wird.

6. Verfahren nach Anspruch 3 oder 5, **dadurch gekennzeichnet, dass** das Verdampfen durch Erzeugen eines Vakuums durchgeführt oder unterstützt wird.

7. Verfahren nach Anspruch 1, **dadurch gekennzeichnet, dass** die zumindest eine Behandlungschemikalie in den Behandlungsbereich (3) vernebelt wird, oder dass die zumindest eine Behandlungschemikalie vor dem Einbringen in den Behandlungsbereich (3) mittels einer Aerosolierungsvorrichtung (46) vernebelt wird.

8. Verfahren nach einem oder mehreren der vorhergehenden Ansprüche, **dadurch gekennzeichnet, dass** eine Menge der zumindest einen Behandlungschemikalie, die in den Behandlungsbereich (3) eingebracht wird, mittels zumindest einer Messvorrichtung (34) gesteuert wird.

9. Verfahren nach einem oder mehreren der vorhergehenden Ansprüche, **dadurch gekennzeichnet, dass** die zumindest eine Behandlungschemikalie während eines zweiten Betriebszustands in den Behandlungsbereich (3) eingebracht wird, wobei im zweiten Betriebszustand das Fördern der Behältnisse (2) und die Abgabe der temperaturgeregelten Prozessflüssigkeit auf die Behältnisse (2) gestoppt wird oder wurde.

10. Verfahren nach Anspruch 1, **dadurch gekennzeichnet, dass** Behältnisse (2), die auf den Außenseiten Aluminium umfassen, durch den Behandlungsbereich (3) gefördert werden.

11. Verfahren nach Anspruch 1, **dadurch gekennzeichnet, dass** die zumindest eine Behandlungschemikalie, die in den Behandlungsbereich (3) eingebracht wird, Ameisensäure oder Essigsäure umfasst.

12. Verfahren nach einem oder mehreren der vorhergehenden Ansprüche, **dadurch gekennzeichnet, dass** zumindest im ersten Betriebszustand, pro Zeiteinheit eine Teilmenge der Prozessflüssigkeit fortlaufend aus dem geschlossenen Kreislauf (14) entnommen wird und mittels einer Membranfiltrationsvorrichtung (50) und in Folge mittels einer Ionenaustauschvorrichtung (51) und/oder mittels einer Adsorptionsvorrichtung (52) gereinigt wird, und die gereinigte Prozessflüssigkeit in den Behandlungsbereich (3) zurück geführt wird.

## Revendications

1. Procédé pour faire fonctionner un dispositif de pasteurisation (1) comprenant un premier état de fonctionnement, dans lequel
dans le premier état de fonctionnement, des récipients scellés (2) remplis d'un produit alimentaire sont transportés à travers une zone de traitement (3) comprenant au moins une zone de chauffage (6, 7),
le produit alimentaire est chauffé et pasteurisé dans la au moins une zone de chauffage (6, 7) en distribuant un liquide de traitement à température commandée sur les récipients (2),
dans lequel au moins une majeure partie du liquide de traitement provenant de la zone de traitement (3) est renvoyée à la zone de traitement (3) dans un circuit en boucle fermée (14) pour être réutilisée,
et dans lequel dans le premier état de fonctionnement, des récipients scellés (2) comprenant un matériau métallique sur une partie extérieure des récipients (2) sont transportés à travers la zone de traitement (3),
caractérisé en ce
au moins un produit chimique de traitement pour un traitement de récipient est introduit dans la zone de traitement (3) à l'état gazeux ou en aérosol au moyen d'un dispositif d'alimentation (21) dans n'importe quel état de fonctionnement du dispositif de pasteurisation (1),
dans lequel le au moins un produit chimique de traitement introduit dans la zone de traitement (3) comprend un acide carboxylique volatil.

2. Procédé selon la revendication 1, **caractérisé en ce que** le au moins un produit chimique de traitement est introduit dans la zone de traitement (3) au moyen d'un écoulement de gaz porteur.

3. Procédé selon la revendication 1 ou 2, **caractérisé en ce que** le au moins un produit chimique de traitement est vaporisé avant introduction dans la zone de traitement (3).

4. Procédé selon la revendication 3, **caractérisé en ce que** le au moins un produit chimique de traitement est évaporé dans un gaz porteur avant introduction dans la zone de traitement (3), et est ensuite introduit dans la zone de traitement (3) au moyen du gaz porteur.

5. Procédé selon la revendication 3, **caractérisé en ce que** la vaporisation est conduite ou supportée au moyen d'un dispositif de chauffage (33).

6. Procédé selon la revendication 3 ou 5, **caractérisé en ce que** la vaporisation est effectuée ou supportée au moyen d'une application d'un vide.

7. Procédé selon la revendication 1, **caractérisé en ce que** le au moins un produit chimique de traitement est nébulisé dans la zone de traitement (3), ou **en ce que** le au moins un produit chimique de traitement est nébulisé avant introduction dans la zone de traitement (3) au moyen d'un dispositif d'aérosolisation (46).

8. Procédé selon l'une quelconque des revendications précédentes, **caractérisé en ce qu'**une quantité du au moins un produit chimique de traitement introduit dans la zone de traitement (3) est commandée au moyen d'au moins un dispositif de dosage (34).

9. Procédé selon l'une quelconque des revendications précédentes, **caractérisé en ce que** le au moins un produit chimique de traitement est introduit dans la zone de traitement (3) pendant un second état de fonctionnement, dans lequel, dans le second état de fonctionnement, le transport des récipients (2) et la distribution de liquide à température commandée sur les récipients (2) sont ou ont été arrêtés.

10. Procédé selon la revendication 1, **caractérisé en ce que** des récipients (2) comprenant de l'aluminium sur l'extérieur sont transportés à travers la zone de traitement (3).

11. Procédé selon la revendication 1, **caractérisé en ce que** le au moins un produit chimique de traitement introduit dans la zone de traitement (3) comprend de l'acide formique ou de l'acide acétique.

12. Procédé selon l'une quelconque des revendications précédentes, **caractérisé en ce qu'**au moins dans le premier état de fonctionnement, une quantité partielle du liquide de traitement est extraite en continu du circuit en boucle fermée (14) par unité de temps, et est purifiée au moyen d'un dispositif de filtration à membrane (50) et ensuite au moyen d'un dispositif d'échange d'ions (51) et/ou au moyen d'un dispositif d'adsorption (52), et le liquide de traitement purifié est renvoyé dans la zone de traitement (3).
